Europäisches Patentamt

European Patent Office

Office européen des brevets

(19)

(11) Numéro de publication : **0 437 586 B1**

(12) **FASCICULE DE BREVET EUROPEEN**

(45) Date de publication du fascicule du brevet :
**02.02.94 Bulletin 94/05**

(51) Int. Cl.$^5$ : **C07D 487/22,** B01J 31/22,
C07D 257/00, C07D 209/00

(21) Numéro de dépôt : **90912571.8**

(22) Date de dépôt : **07.08.90**

(86) Numéro de dépôt international :
**PCT/FR90/00600**

(87) Numéro de publication internationale :
**WO 91/01985 21.02.91 Gazette 91/05**

(54) **MESO-TETRAPHENYLE PORPHYRINES PHENYLE SUBSTITUEES.**

(30) Priorité : **10.08.89 FR 8910762**

(43) Date de publication de la demande :
**24.07.91 Bulletin 91/30**

(45) Mention de la délivrance du brevet :
**02.02.94 Bulletin 94/05**

(84) Etats contractants désignés :
**AT BE CH DE DK ES FR GB IT LI LU NL SE**

(56) Documents cités :
**J.Org.Chem., vol. 54, 17 fevr. 1989, American
Chemical Society, Washington, US, J.S. Lindsey et al.: "Investigation of the synthesis of
ortho-substituted tetraphenylporphyrins",
pages 828-836, voir page 832, tableau V**

(73) Titulaire : **SOCIETE NATIONALE ELF
AQUITAINE
Tour Elf, 2, Place de la Coupole, La Défense 6
F-92400 Courbevoie (FR)**
Titulaire : **ELF ATOCHEM S.A.
4 & 8, Cours Michelet La Défense 10
F-92800 Puteaux (FR)**

(72) Inventeur : **MEUNIER, Bernard
Résidence Les Ormes Bât. C3
F-31320 Castanet (FR)**
Inventeur : **LABAT, Gilles
45, rue George-Sand
F-31400 Toulouse (FR)**
Inventeur : **HOFFMANN, Pascal
5, rue Georges-Bidault Appt. 40
F-31400 Toulouse (FR)**
Inventeur : **SERIS, Jean-Louis Lotissement
Parenche No 15
Chemin Vignats
F-64110 Jurançon (FR)**

(74) Mandataire : **Ohresser, François et al
Société Nationale Elf Aquitaine Division
Proprieté Industrielle Tour Elf
F-92078 Paris La Défense Cédex 45 (FR)**

EP 0 437 586 B1

EP 0 437 586 B1

## Description

La présente invention a pour objet de nouveaux composés hydrosolubles de la porphyrine et leurs dérivés métalliques.

Depuis une dizaine d'années de nombreuses études sur l'utilisation de métalloporphyrines synthétiques solubles en milieu organique comme catalyseurs d'oxydation des hydrocarbures ont été engagées.

Ces composés appartiennent généralement à la famille des méso-tétraphényl porphyrines. L'efficacité de ces catalyseurs dépend en partie de la nature des substituants des groupes phényles liés aux positions méso (5, 10, 15, 20) de la porphyrine. En particulier la présence de substituants en ortho et ortho' (2,6) de ces phényles permet, grâce à un effet cage, d'éviter la formation en milieu oxydant d'entités $\mu$-oxo, c'est-à-dire deux cycles porphyriniques liés entre eux via le métal par un atome d'oxygène, inertes en catalyse ou même la destruction du catalyseur par transfert intermoléculaire d'espèce oxo (J.T. Groves et T.E. Nemo, J. AM. Chem. Soc, 105, 6243.6248 ; 1983)

Deux de ces complexes sont plus largement utilisés : les dérivés fer (III) et manganèse (III) de la méso-tétramésityl porphyrine, (M-TMP) et de la méso-tétrakis (2,6-dichloro-phényle porphyrine (M-TDCPP).

La synthèse de ces porphyrines encombrées est difficile selon les méthodes classiques. Récemment il a été proposé une synthèse par oxydation du porphyrinogène (R.W. Wagner, J.S. Lindsey et al., Tetrahedron. Lett, 28, 3069-3070 ; 1987) selon laquelle on fait réagir un aldéhyde sur le cycle pyrrole pour obtenir le macrocycle porphyrinique substitué en méso.

Il est par ailleurs important pour que ces complexes puissent être utilisés comme catalyseurs en milieu liquide comportant de l'eau, de leur conférer un caractère hydrosoluble. Jusqu'à présent peu de travaux ont décrit l'utilisation des porphyrines encombrées comme ligands de métalloporphyrines hydrosolubles. On peut juste relever la méso-tétrakis (2,6 dimethyl-3-sulfonatophényle) porphyrine décrite par T.C. Bruice et coll., J. Am. Chem. Soc., 109, 7865-7873, (1987).

Il faut cependant observer que l'aldéhyde aromatique précurseur de cette porphyrine est peu accessible ce qui limite considérablement l'intérêt de ce complexe comme catalyseur.

La présente invention propose de nouvelles méso-tétraphényle porphyrines hydrosolubles substituées, faciles à préparer et dont les dérivés métalliques sont robustes en milieu oxydant et donc efficaces comme catalyseurs d'oxydation des hydrocarbures.

La présente invention a pour objet des méso-tétraphényle porphyrines caractérisées en ce que tous les carbones libres des groupements phényles sont substitués en positions 2, 4, 6 par un reste hydrocarboné et en positions 3, 5 par une fonction sulfonate.

Les restes hydrocarbonés placés en position 2, 4, 6 des groupements phényles sont tous identiques. Ils seront soit des groupes alkyles comportant de 1 à 6 atomes de carbone : méthyle, éthyle, propyle..., soit des groupes aryles, soit des groupes fonctionnels tels que les radicaux trifluorométhyle, méthoxyle.

On utilisera de préférence, essentiellement pour des raisons de disponibilité et donc de coût des produits permettant la synthèse, le méthyle comme substituant des carbones 2, 4, 6 des groupements phényles.

Il va de soi que l'invention vise également les porphyrines substituées sur les noyaux pyrroles telles que par exemple les tétra($\beta$-dibromopyrrole) porphyrines.

L'invention a également pour objet les dérivés complexes métalliques de ces méso-tétraphényle porphyrines substituées et notamment les complexes fer (III) et manganèse (III), qui pourront être utilisés comme catalyseurs d'oxydation des hydrocarbures.

L'invention a également pour objet un procédé de préparation de ces méso-tétraphényle porphyrine substituées.

Ce procédé consiste:
- dans une première étape à préparer selon un processus connu le benzaldehyde tri-substitué en positions 2, 4, 6 par le reste hydrocarboné choisi.
- dans une deuxième étape à faire réagir cet aldéhyde sur le pyrrole selon le processus décrit par R.W. Wagner ci-dessus référencé pour obtenir la méso-tétra (2, 4 , 6 tri-substitué phényle) porphyrine,
- dans une troisième étape à sulfoner cette porphyrine dans des conditions dures en la traitant par du $SO_3$ pur ou par de l'oléum, acide sulfurique contenant de 5 à 80 % et de préférence entre 20 et 40 % poids de $SO_3$, à chaud c'est-à-dire à une température comprise entre 40 et 140°C.

La durée de la réaction dépendra de la teneur en $SO_3$ de l'oléum et de la température de mise en oeuvre. Ces conditions dures permettent de diminuer la durée de l'étape de sulfonation. Elle sera en général inférieure à 5 heures, et pourra même atteindre quelques minutes en utilisant du $SO_3$ pur. Il faudra, en outre, veiller au cours de cette étape à ne pas détruire le macrocycle porphyrinique.

La porphyrine ainsi obtenue peut être ensuite métallée par le fer ou le manganèse selon un processus connu décrit par R.D. Jones et Coll. J. Am. Chem. Soc., 1978, 100, 4416.

2

L'invention sera mieux comprise à la lecture des exemples ci-après qui illustrent :
- la synthèse des méso-tétraphényle porphyrines substituées (exemples 1 et 2),
- la métallation de ces porphyrines (exemples 3 à 6),
- l'utilisation de ces porphyrines comme catalyseurs d'oxydation d'alcools benzyliques (exemples 7 à 14)

EXEMPLE 1 :

Préparation de la méso-tétra-mésitylporphyrine sulfonée (TMPS).

Cette synthèse s'effectue à partir de la TMP produit déjà connu.

Dans un ballon de 100 ml, on dissout 500 mg de TMP ($6.4 \times 10^{-4}$ mol) dans 50 ml d'oléum (acide sulfurique fumant contenant 20 % de $SO_3$ ; Aldrich) . On porte le mélange à 120°C pendant 4 à 5 heures. Après avoir dilué 2 fois avec de l'eau le mélange réactionnel, le pH du milieu est amené à 8-9 par addition d'une solution de soude 2M. L'élimination des sels présents en solution s'effectue par précipitation en additionnant 3 volumes de méthanol. Le mélange obtenu est filtré (cette opération est répétée 2 à 3 fois).

Le filtrat est concentré, puis séché sous vide. Le résidu est repris par un minimum de méthanol puis chromatographié (gel de silice 60 ; 70-230 mesh, éluant méthanol). Après évaporation à sec sous pression réduite, on reprend le produit brut dans le minimum d'eau et celui-ci est précipité par addition d'éthanol. 820 mg de produit (sel octasodique) sont isolés, rendement = 80 %.

EXEMPLE 2 :

Préparation de la $Br_8$ TMPS.

1ère étape :

Synthèse de la 5, 10, 15, 20 - tétramésityl-tétra (β-dibromopyrrole) porphyrine, $Br_8$TMP

La bromation s'effectue sur la TMP métallée par le zinc, ZnTMP. La métallation par le zinc est réalisée de la manière suivante : 740 mg (0.95 mmol) de TMP sont dissout dans 100 ml de diméthylformamide, et le mélange est porté à reflux. On ajoute 5 équivalents de Zn $(OAc)_2$,2 $H_2O$ (1.04g, 4.75 mmole) et 10 équivalents de 2, 4, 6-collidine. Le mélange est maintenu au reflux pendant 1 à 2 heures. En fin de réaction, on verse le milieu préalablement ramené à température ambiante dans 100 ml de dichlorométhane, ZnTMP précipite. Après filtration et séchage sous vide, on obtient 650 mg, rendement = 81 %. La bromation s'effectue selon le protocole suivant : on ajoute à une solution de N-bromosuccinimide (2g dans 200 ml de dichlorométhane) 500 mg de ZnTMP (0.59 mmole) , et le mélange est agité à température ambiante pendant 12 heures. On neutralise le milieu réactionnel par 200 ml d'une solution de NaOH 0.1 M. La phase organique est séchée sur sulfate de magnésium, évaporée. Le produit obtenu est chromatographié sur alumine activée 90 basique (70-230 mesh Merck, éluant : gradient hexane/dichlorométhane 90/10, v/v puis 50/50, v/v). On obtient 532 mg de $ZnBr_8$TMP, Rendement = 61 %. La porphyrine est démétallée par l'acide trifluoroacétique (10 % en solution dans le dichlorométhane).

La réaction est instantanée. Après neutralisation par une solution de bicarbonate de sodium saturée, puis lavages à l'eau, la solution organique est évaporée à sec, et le produit est chromatographié sur alumine activée 90 basique (70-230 mesh Merck, éluant dichlorométhane). On obtient 453 mg de $Br_8$TMP, rendement = 89 %.

2ème étape :

Synthèse de la 5, 10, 15, 20 tétrakis-(3,5-disulfonatomésityl)-tétra (β-dibromopyrrole) porphyrine, $Br_8$TMPS.

La sulfonation se fait suivant le même protocole que celui décrit pour la synthèse de la TMPS à partir de la TMP. Rendement de cette étape : 75 %.

EXEMPLE 3

Préparation de la Mn-TMPS, dérivé du manganèse (III) de la tétra (3,5-disulfonatomésityl) porphyrine.

Cinq équivalents de Mn(OAc)$_2$, 4H$_2$O (300 mg, 1.22 mmol), dix équivalents de 2, 4, 6-collidine (320 μl, 2.44 mmol) sont additionnés à une solution de la porphyrine TMPS (390 mg, 0.244 mmole dans 30 ml de diméthylformamide). Le mélange est porté à 140°C pendant 1 à 2 heures. La purification s'effectue de la manière suivante : on additionne du méthanol au mélange réactionnel afin de précipiter les sels. Après filtration et évaporation à sec, sous pression réduite à chaud (0.1 mm Hg, 100°C), le résidu est repris dans de l'eau. Cette solution est passée sur une résine échangeuse de cations AG 50 W-X8 200-400 mesh (Bio-Rad) préalablement lavée à l'eau. Après avoir amené le pH de cette solution à 6.0 avec une solution de soude 1 M, on évapore sec puis on reprend avec le minimum de méthanol, on effectue alors une purification sur gel de silice 60 (70-230 mesh Merck, éluant méthanol). 317 mg du sel de sodium de Mn-TMPS sont isolés ; Rdt = 76 %.

EXEMPLE 4 :

Préparation de la Fe-TMPS : dérivé du fer (III) de la tétra (3, 5-disulfonatomésityl) porphyrine.

Cinq équivalents de sel de Mohr, $Fe(NH_4)_2(SO_4)_2$-$6H_2O$(478 mg, 1.22 mmol), 10 équivalents de 2, 4 , 6-collidine (320 µl, 2.44 mmol) sont additionnés à une solution de porphyrine TMPS (390 mg, 2.44 mmole dans 30 à 40 ml de diméthylformamide). Le pH est maintenu entre 5 et 8 durant la métallation par une solution de soude 1M. Le mélange réactionnel est porté à 140°C pendant 12 heures. La purification s'effectue suivant une procédure analogue à celle décrite précédemment pour Mn-TMPS. La métallation peut se faire également avec $FeCl_2$, $2H_2O$ et $Fe(OAC)_2$ anhydre. 284 mg du sel de sodium sont isolés, Rdt = 60 %.

EXEMPLE 5 :

Préparation de la Mn-$Br_8$TMPS, dérivé du manganèse (III) de la 5, 10, 15, 20-tétrakis-(3,5-disulfonatomésityl)-(tétra (β-dibromopyrrole)porphyrine, $Br_8$TMPS.

Dix équivalents de $Mn(OAc)_2$, $4H_2O$(175 mg, 0.72 mmol) , dix équivalents de 2, 4, 6 - collidine (95 µl, 0.72 mmol) sont additionnés à une solution de porphyrine $Br_8$TMPS (160 mg dans un mélange diméthylformamide/eau, 90/10, v/v). Le mélange est porté à 110°C pendant 1 heure. La purification s'effectue de la manière suivante : on évapore à sec le mélange réactionnel puis on additionne du méthanol. Après filtration et évaporation à sec, sous pression réduite à chaud (0.1 mm Hg, 100°C), le résidu est repris dans de l'eau. Cette solution est passée sur une résine échangeuse de cations AG 50 W-X8 200-400 mesh (Bio-Rad) préalablement lavée à l'eau. Après avoir amené le pH de cette solution à 6.0 avec une solution de soude 1M, on évapore à sec puis on reprend avec le minimum de méthanol, on effectue alors une purification sur gel de silice 60 (70-230 mesh Merck, éluant : dichlorométhane/méthanol, 50/50, v/v). La porphyrine dissoute dans le minimum d'eau, est ensuite précipitée par addition d'acétone. On effectue alors un lavage par un mélange acétone/eau, 90/10, v/v. 120 mg du sel de sodium de Mn-$Br_8$TMPS sont isolés ; Rdt = 72 %.

EXEMPLE 6 :

Préparation de la Fe-$Br_8$TMPS : dérivé du fer (III) de la 5, 10, 15, 20-tétrakis-(3,5-disulfonatomésityl)-tétra (β-dibromopyrrole) porphyrine, $Br_8$TMPS.

Dix équivalents de $FeCl_2$, 4 $H_2O$ (88.5 mg, 0.44 mmol) et 20 équivalents de 2, 4, 6-collidine (115 µl, 0.894 mmol) sont additionnés à une solution de porphyrine $Br_8$TMPS (100 mg, 44 µmol dans 20 ml de diméthylformamide). Le pH est maintenu entre 5 et 8 durant la métallation par une solution de soude 1M. Le mélange réactionnel est porté à 160°C pendant 2 heures. On rajoute de nouveau dix équivalents de Fe $Cl_2$, 4 $H_2O$ (88.5 mg, 0.44 mmol) et 20 équivalents de 2, 4, 6-collidine (115 µl, 0.894 mmol) afin de réaliser une métallation complète pendant 30 minutes. On évapore à sec le mélange réactionnel puis on additionne du méthanol. Après filtration et évaporation à sec, sous pression réduite à chaud (0.1 mm Hg, 100°C), la métalloporphyrine est reprise dans le minimum d'eau. On additionne 2 volumes d'acétone afin de précipiter Fe-$Br_8$TMPS, 62 mg sont isolés : rendement = 60 %.

EXEMPLES 7 à 14 :

Ces exemples illustrent l'utilisation des porphyrines de l'invention comme catalyseur d'oxydation d'alcool vératrylique (substrat I) et du 1-(3,4 -diméthoxyphényl) 2-(2-méthoxyphénoxy) propane-1,3-diol(substrat II).

Les catalyseurs M-TMPS sont utilisés, soit sous forme libre en catalyse homogène, soit sous forme supportée après fixation par imprégnation sur une résine échangeuse d'ions (Amberlite, IRA 900) selon la méthode de Y. Saito et Coll (Pure and Appl. Chem., 59, 573, 1987). Cette fixation est symbolisée par le suffixe -Ad

Conditions générales des essais catalytiques.

La conversion des substrats I ou II transformés lors de la réaction catalytique est mesurée par analyse HPLC.

La solution oxydante utilisée est, sauf indication contraire, pour les exemples comparatifs, à base d'hydrogénopersulfate de potassium : KHSO₅.
- Dans le cas de catalyse homogène utilisant le M-TMPS libre (M = Fe, Mn), le substrat, 20µmol dans 500 µl d'acétonitrile et le catalyseur M-TMPS, 2 µmol dans 1 ml de tampon citrate
- phosphate 0,1 M ; pH = 3.0 pour le fer et tampon phosphate 0,5 M ; pH = 6.0 pour le manganèse, sont préalablement mélangés.

L'addition de la solution oxydante, 100 µmol d'Oxone [R] : 2KHSO₅, KHSO₄, K₂SO₄, 30,7 mg déclenche la réaction catalytique menée à la température ambiante.
- Dans le cas de la catalyse supportée utilisant le M-TMPS-Ad (M = Fe, Mn) le catalyseur M-TMPS-Ad (100 mg) est ajouté à la solution de substrat, 20 µmol dans 1,5 ml de solution acétonitrile-tampon, pH = 3.0 (1/1, v/v). La réaction catalytique est également déclenchée par l'addition de la solution oxydante.

L'addition de la base azotée, 4-tert-butylpyridine, en excès, 100 équivalents par rapport au catalyseur, est effectuée lors de la dissolution du substrat dans l'acétonitrile.

Conditions particulières

EXEMPLE 7 :

Substrat I (20 µmol, 500 µl d'une solution d'acétonitrile à 40 mM en alcool vératrylique) ; FeTMPS (200 nmol ; 100 µl d'une solution de porphyrine de fer à 2mM tamponnée pH 3.0); tampon citrate-phosphate 0.1 M (900 µl) KHSO₅ (100 µmol; 30.7 mg dissous dans 500 µl de tampon pH 3.0).

EXEMPLE 8 :

Mêmes conditions expérimentales que pour l'exemple 7. Remplacement de Fe-TMPS par Fe-TMPS-Ad.

EXEMPLE 9 :

Substrat I (20 µmol ; 500 µl d'une solution d'acétonitrile à 40mM en alcool vératrylique) ; Mn-TMPS (2 µmol ; 3.2 mg dissous dans 500 µl de tampon 0.5 M pH 6.0) ; 4-tert-butylpyridine (200 µmol ; 28 µl) ; KHSO₅ (100 µmol ; 30.7 mg dissous dans 500 µl de tampon phosphate 0.5 M pH 6.0).

EXEMPLE 10 :

Substrat I (20 µmol ; 500 µl d'une solution d'acétonitrile à 40 mM) ; Mn-TMPS-Ad (2 µmol ; 100 mg) ; tampon phosphate pH 6.0 (1 ml); 4-tert-butyle pyridine (200 µmol, 28 µl) ; KHSO₅ (100 µmol ; 30.7 mg dissous dans 500 µl tampon phosphate 0.5 M pH 6.0).

EXEMPLE 11 :

Mêmes conditions expérimentales que pour l'exemple 7. Remplacement du substrat I par le substrat II.

EXEMPLE 12 :

Mêmes conditions expérimentales que pour l'exemple 8. Remplacement du substrat I par le substrat II.

EXEMPLE 13 :

Mêmes conditions expérimentales que pour l'exemple 9. Remplacement du substrat I par le substrat II.

EXEMPLE 14 :

Mêmes conditions expérimentales que pour l'exemple 10. Remplacement du substrat I par le substrat II.
Les résultats obtenus sont résumés dans le tableau 1.

EP 0 437 586 B1

TABLEAU 1

| Exemple n° | catalyseur | % cata/ substrat (molaire) | pH | % conversion après (min) | |
|---|---|---|---|---|---|
| | | | | 1 | 15 |
| 7 | Fe-TMPS | 1 | 3,0 | 100 | – |
| 8 | Fe-TMPS-Ad | 10 | 3,0 | 100 | – |
| 9 | Mn-TMPS | 10 | 6,0 | 63 | 100 |
| 10 | Mn-TMPS-Ad | 10 | 6,0 | 74 | 82 |
| 11 | Fe-TMPS | 1 | 3,0 | 66 | 90 |
| 12 | Fe-TMPS-Ad | 10 | 3,0 | 40 | 65 |
| 13 | Mn-TMPS | 10 | 6,0 | 76 | 100 |
| 14 | Mn-TMPS-Ad | 10 | 6,0 | 46 | 60 |

**Revendications**

1. Méso-tétraphényle porphyrines hydrosolubles caractérisées en ce que tous les carbones libres des groupements phényles sont substitués en positions 2, 4, 6 par un reste hydrocarboné et en positions 3, 5 par une fonction sulfonate.

2. Méso-tétraphényle porphyrines de la revendication 1 caractérisées en ce que le reste hydrocarboné est un radical alkyle, comportant de 1 à 6 atomes de carbone.

3. Mesotétraphényl porphyrine de la revendication 2 caractérisées en ce que le reste hydrocarboné est le radical méthyle.

4. Procédé de préparation des méso-tétraphényle porphyrines des revendications 1 à 3 caractérisé en ce que :
   - dans une première étape on prépare un aldéhyde benzylique trisubstitué en positions 2, 4, 6,
   - dans une deuxième étape on fait réagir l'aldéhyde issu de la première étape avec le pyrrole pour obtenir la méso-tétra (2, 4, 6 - trisubstitué phényle) porphyrine,
   - dans une troisième étape on sulfone la porphyrine issue de la deuxième étape en la traitant par de l'oléum, acide sulfurique contenant de 5 à 80 % et du $SO_3$ ou de préférence 20 à 40 % poids de $SO_3$ à une température comprise entre 40°C et 140°C.

5. Utilisation des meso-tétraphényl porphyrines des revendications 1 à 3, sous forme métallée, fer III ou manganèse III, comme catalyseurs d'oxydation des hydrocarbures.

6

**Patentansprüche**

1. Wasserlösliche m-Tetraphenylporphyrine, dadurch **gekennzeichnet**, daß die freien C-Atome der Phenylgruppen in 2-, 4- und 6-Stellung durch einen Kohlenwasserstoffrest und in 3- und 5-Stellung durch eine funktionelle Sulfonatgruppe substituiert sind.

2. m-Tetraphenylporphyrine nach Anspruch 1, dadurch **gekennzeichnet,** daß der Kohlenwasserstoffrest ein 1 bis 6 C-Atome enthaltender Alkylrest ist.

3. m-Tetraphenylporphyrin nach Anspruch 2, dadurch **gekennzeichnet**, daß der Kohlenwasserstoffrest der Methylrest ist.

4. Verfahren zur Herstellung der m-Tetraphenylporphyrine nach den Ansprüchen 1 bis 3, dadurch **gekennzeichnet**, daß man:
   - auf einer ersten Stufe einen in 2-, 4-, 6-Stellung trisubstituierten Benzaldehyd herstellt,
   - auf einer zweiten Stufe, den auf der ersten Stufe erhaltenen Aldehyd mit dem Pyrrol zum m-Tetra-(2, 4, 6-trisubstituiertes Phenyl)porphyrin umsetzt und
   - auf einer dritten Stufe das auf der zweiten Stufe erhaltene Porphyrin sulfoniert, indem man es mit Oleum oder Schwefelsäure mit 5 bis 80 und vorzugsweise 20 bis 40 Gew.-% $SO_3$ bei einer Temperatur zwischen 40 und 140°C behandelt.

5. Verwendung der m-Tetraphenylporphyrine nach Anspruch 1 bis 3 in mit Fe(III) oder Mn(III) metallierter Form als Katalysatoren für die Oxydation von Kohlenwasserstoffen.


**Claims**

1. Hydrosoluble meso-tetraphenyl porphyrins, characterised in that all the free carbon atoms of the phenyl groups are substituted by a hydrocarbon residue in positions 2, 4, 6 and by a sulphonate functional group in positions 3, 5.

2. Meso-tetraphenyl porphyrins of Claim 1, characterised in that the hydrocarbon residue is an alkyl radical comprising from 1 to 6 carbon atoms.

3. Meso-tetraphenyl porphyrin of Claim 2, characterised in that the hydrocarbon residue is the methyl radical.

4. Method of preparing the meso-tetraphenyl porphyrins of Claims 1 to 3, characterised in that:
   - in a first step, a benzyl aldehyde, trisubstituted in positions 2, 4, 6, is prepared;
   - in a second step the aldehyde produced in the first step is made to react with pyrrole to produce meso-tetra (2, 4, 6-trisubstituted phenyl) porphyrin;
   - in a third step the porphyrin produced in the second step is sulphonated by being treated with oleum, sulphuric acid containing from 5 to 80% of $SO_3$ or, preferably, from 20 to 40% by weight of $SO_3$, at a temperature of between 40°C and 140°C.

5. Use of the meso-tetraphenyl porphyrins of Claims 1 to 3 in the iron III or manganese III metallic form, as catalysts for the oxidation of hydrocarbons.